**Europäisches Patentamt**

**European Patent Office**

**Office européen des brevets**

(19)

(11) Numéro de publication : **0 164 780**
**B1**

(12)

# FASCICULE DE BREVET EUROPÉEN

(45) Date de publication du fascicule du brevet :
15.03.89

(51) Int. Cl.⁴ : **G 01 N 33/74//** G01N33/546,
G01N33/563

(21) Numéro de dépôt : **85200745.9**

(22) Date de dépôt : **10.05.85**

(54) Procédé de dosage immunologique d'une substance dans un échantillon liquide.

(30) Priorité : **15.05.84 BE 212939**

(43) Date de publication de la demande :
**18.12.85 Bulletin 85/51**

(45) Mention de la délivrance du brevet :
**15.03.89 Bulletin 89/11**

(84) Etats contractants désignés :
**AT CH DE FR GB IT LI LU NL SE**

(56) Documents cités :
EP--A-- 0 038 181
EP--A-- 0 051 985
FR--A-- 2 502 787
GB--A-- 2 051 081

(73) Titulaire : **l'Association internationale à but scientifique, dite: Institut international de pathologie cellulaire et moléculaire**
**Avenue Hippocrate 75**
**B-1200 Woluwé-Saint-Lambert (BE)**

(72) Inventeur : **Mareschal, Jean-Claude**
**Avenue de la Vècquée 196**
**B-5730 Malonne (BE)**
Inventeur : **Masson, Pierre**
**Avenue Emile Vandervelde 107**
**B-1200 Bruxelles (BE)**

(74) Mandataire : **Schmitz, Yvon et al**
**Bureau Gevers S.A. rue de Livourne 7 bte 1**
**B-1050 Bruxelles (BE)**

Jouve, 18, rue St-Denis, 75001 Paris, France

# EP 0 164 780 B1

**Description**

La présente invention est relative à un procédé de dosage immunologique d'une substance choisie dans un échantillon liquide, par exemple un liquide biologique contenant une ou plusieurs autres substances non choisies, susceptibles d'interférer dans le dosage de la substance choisie.

Comme on le sait, certaines substances, telles que les hormones animales et humaines, comportent au moins deux chaînes ou fragments structuralement différents, faiblement liés l'un à l'autre, l'une de ces chaînes ou l'un de ces fragments étant immunologiquement commun et l'autre immunologiquement distinct pour chaque substance. Le dosage de ces substances s'avère souvent particulièrement important pour ce qui est du diagnostic mais celui-ci est extrêmement délicat à réaliser, des réactions croisées apparaissant souvent entre la substance à doser et les autres substances, qui interfèrent par conséquent dans le dosage de cette substance, et qui faussent celui-ci. Bien que l'on puisse induire un antisérum hautement spécifique de la substance à doser, en utilisant uniquement la chaîne ou le fragment structuralement distinct de cette dernière, il subsiste toujours une certaine réactivité croisée, qui fausse le dosage de la substance concernée. Toutefois, cette réactivité peut encore être réduite en traitant l'antisérum par la molécule ou substance qui se rapproche structuralement le plus de la substance à doser vers laquelle est dirigé cet antisérum. Les anticorps contenus dans l'antisérum qui ne sont pas totalement spécifiques de la substance à doser se lieront alors à l'excès de cette autre substance et peuvent être séparés de l'antisérum. Une telle technique est efficace et donne des résultats relativement bons, notamment lorsque la substance de traitement est, par exemple, la gonadotropine chorionique humaine (hCG), dont le coût n'est pas prohibitif. Toutefois, lorsqu'il s'agit d'utiliser des composés tels que l'hormone folliculostimulante (FSH), l'hormone lutéinisante (LH) ou la thyréostimuline (TSH), ce procédé n'est plus intéressant, le coût de ces substances étant prohibitif.

L'objet de la présente invention consiste, par conséquent, à pallier les inconvénients des procédés de dosage connus et à prévoir un procédé permettant d'améliorer la spécificité d'un dosage immunologique d'une substance choisie dans un liquide, tel qu'un liquide biologique contenant une ou plusieurs autres substances non choisies, susceptibles d'interférer dans le dosage de la substance choisie, sans qu'il s'avère nécessaire d'utiliser une substance purifiée présentant une réaction croisée, dont le coût, dans la plupart des cas, est extrêmement élevé. Le procédé de l'invention peut être utilisé pour le dosage de n'importe quel type de substance, pour autant que cette substance et la ou les autres substances susceptibles d'interférer dans le dosage soient formées d'au moins deux chaînes ou fragments structuralement différents, l'une de ces chaînes ou l'un de ces fragments étant immunologiquement commun et l'autre immunologiquement distinct.

A cet effet, suivant l'invention, on ajoute un agent dissociant choisi dans le groupe comprenant les agents protéolytiques, les agents rompant les ponts disulfure, les agents chaotropiques et les mélanges d'au moins deux de ces agents à l'échantillon liquide pour scinder la substance choisie à doser en au moins ses deux chaînes ou fragments, la ou les substances non choisies étant également scindées en au moins ses ou leurs deux chaînes ou fragments, on mélange le liquide ainsi obtenu, on inactive l'agent dissociant, on ajoute audit mélange un antisérum dirigé contre la chaîne ou le fragment immunologiquement commun de la substance choisie et de la ou des substances non choisies, on ajoute à la solution résultante une suspension de particules recouvertes d'un antisérum dirigé contre la chaîne ou le fragment immunologiquement distinct de la substance choisie à doser et on détermine la quantité de cette substance choisie dans l'échantillon liquide par une technique d'agglutination de particules précitées.

Suivant une forme de réalisation particulière du procédé de l'invention, la substance choisie et la ou les substances non choisies sont des hormones, telles que la thyréostimuline (TSH), l'hormone folliculostimulante (FSH), l'hormone lutéinisante (LH) et la gonadotropine chorionique humaine (hCG), les chaînes immunologiquement commune et distincte étant constituées dans ce cas respectivement par les chaînes alpha et bêta.

Des exemples d'agents protéolytiques sont les enzymes protéolytiques, telles que la pepsine, la papaïne et la trypsine, la pepsine convenant particulièrement bien à cet effet. Des exemples d'agents rompant les ponts disulfure sont les substances à activité réductrice, telles que le mercaptoéthanol et le dithiotréitol. Des exemples d'agents chaotropiques sont l'urée, le chlorhydrate de guanidine et le thiocyanate d'ammonium.

Suivant l'invention, on détermine la quantité de substance choisie dans l'échantillon liquide par une technique d'agglutination de particules, la quantité de substance choisie étant déterminée en fonction du degré d'agglutination ou de non-agglutination de particules finement divisées, lesdites particules étant de préférence faites d'acrylamide ou de polystyrène ou constituées par des globules rouges. Les particules d'acrylamide, de polystyrène ou de tout autre polymère sont généralement appelées « latex ».

D'autres détails et particularités de l'invention ressortiront de la description donnée ci-après à titre d'exemple non limitatif de quelques formes de réalisation particulières de l'invention.

Comme on l'a déjà signalé précédemment, la présente invention propose un procédé de dosage immunologique d'une substance choisie, telle que, par exemple, une hormone d'origine animale ou humaine dans un liquide biologique contenant une ou plusieurs autres substances non choisies,

2

susceptibles d'interférer dans le dosage de la substance à doser, ces autres substances pouvant également être constituées d'hormones d'origine animale ou humaine, à des fins de diagnostic, sans qu'il n'y ait de réactions croisées faussant le dosage de la substance choisie. Des hormones extrêmement importantes pour un diagnostic et qui sont difficiles à doser sans l'apparition de réactions croisées sont, comme on l'a également déjà signalé précédemment, la thyréostimuline (TSH), l'hormone folliculostimulante (FSH), l'hormone lutéinisante (LH) et la gonadotropine chorionique humaine (hCG). Chacune de ces substances est formée de deux parties, faiblement liées l'une à l'autre appelées chaînes alpha et bêta, la chaîne alpha de ces substances étant immunologiquement commune, la chaîne bêta étant immunologiquement distincte pour chaque substance.

Suivant l'invention, on ajoute au liquide biologique contenant la substance à doser un agent dissociant, tel quel, par exemple, une enzyme protéolytique qui peut être de la pepsine, de la papaïne ou de la trypsine, la substance à doser et la ou les substances susceptibles d'interférer dans le dosage étant brisées par digestion au moyen de cette enzyme en au moins leurs deux chaînes structuralement différentes. Dans le cas des hormones précitées, on pourrait, par exemple, utiliser le procédé de digestion à la pepsine décrit dans la demande de brevet européen n° 51 985, qui scinde les chaînes alpha et bêta de chaque hormone. La digestion à l'enzyme protéolytique doit être telle que la substance choisie ainsi que la ou les substances non choisies doivent au moins être scindées en leurs deux chaînes, la digestion pouvant être toutefois prolongée, de manière à fragmenter d'une manière plus poussée les deux chaînes structuralement différentes. Une fois la scission ou fragmentation des deux chaînes réalisée, on mélange le liquide ainsi obtenu, par exemple pendant une durée de l'ordre de 5 à 15 minutes, on inactive l'enzyme protéolytique, par exemple au moyen d'un agent neutralisant, en élevant le pH du mélange à une valeur tournant aux alentours de 7 à 9. On ajoute ensuite au mélange un antisérum dirigé contre la chaîne immunologiquement commune de la substance choisie et de la ou des substances non choisies, et on ajoute ensuite à la solution résultante un antisérum dirigé contre la chaîne immunologiquement distincte de la substance choisie à doser. On pourrait, par exemple, lier la chaîne alpha au moyen d'un antisérum dirigé contre l'une ou l'autre des quatre hormones citées ci-dessus, qui comportent toutes la même chaîne alpha. On pourrait, par exemple, suivant l'invention, traiter un échantillon de sérum humain avec de la pepsine, qui scinde la molécule d'hCG en ses chaînes alpha et bêta ou en de plus petits fragments d'une manière connue en soi (voir, par exemple, Kohn, L.D. et Winand R.J. Biol. Chem. 16, 6503-6508, 1975), mélanger la solution obtenue pendant une durée de l'ordre de 5 à 15 minutes et, après inactivation de la pepsine, ajouter un antisérum anti-TSH, dont les anticorps réagissent avec la chaîne alpha ou des fragments de celles-ci des hormones que contient l'échantillon de sérum, c'est-à-dire de LH, FSH, hCG et TSH. Si l'on ajoute ensuite un antisérum dirigé contre la chaîne bêta de l'hCG, les seules antigènes libres sont constituées par la chaîne bêta de cette hormone. Par conséquent, la spécificité du dosage de cette hormone est fortement accrue. Après avoir ajouté les antisérums, on incube généralement la solution obtenue sous agitation à une température d'environ 37 °C pendant une durée de l'ordre de 15 à 60 minutes. Etant donné que de grandes quantités d'antisérum peuvent être induites à partir d'une quantité relativement petite de substance pure, le procédé de l'invention est donc un procédé permettant d'accroître sensiblement la spécificité d'un dosage immunologique d'une substance, extrêmement simple à utiliser et économique. On pourrait, bien entendu, ainsi qu'on l'a déjà mentionné précédemment, utiliser d'autres agents que des agents protéolytiques comme agents dissociants, par exemple des agents rompant les ponts disulfure ou des agents chaotropiques, qui ont la fonction de rompre les liaisons hydrogène et hydrophobes. On citera, à cet effet, à titre d'exemples non limitatifs, comme agents rompant les ponts disulfure, les substances à activité réductrice, telles que le mercaptoéthanol, et le dithiotréitol et, comme agents chaotropiques, l'urée, le chlorhydrate de guanidine et le thiocyanate d'ammonium.

Suivant l'invention, on détermine de préférence la quantité de substance choisie dans le liquide biologique, après l'addition des antisérums, par une technique d'agglutination de particules, la quantité de substance choisie étant déterminée en fonction du degré d'agglutination ou de non-agglutination de particules finement divisées qui, comme on l'a déjà mentionné précédemment, sont de préférence des particules d'acrylamide ou de polystyrène ou des globules rouges. On décrira ci-après, à titre d'exemple non limitatif, le dosage de hCG en utilisant la technique d'immunoessai par comptage de particules de latex (PACIA), bien que, comme le savent les spécialistes, cette technique puisse être également appliquée à un grand nombre d'autres types d'immunoessai, tels que les radioimmunoessai, enzymoim-munoessai, fluoroimmunoessai, etc.

La mesure d'hCG est très importante dans le diagnostic d'une grossesse à un stade très précoce. La détermination de la teneur en hCG est également importante pour diagnostiquer rapidement une grossesse ectopique de manière à empêcher une rupture des trompes et d'importantes hémorragies. Enfin, comme marqueur de tumeur, la mesure de la teneur en hCG est souvent le seul test que l'on puisse réaliser pour déterminer la réponse d'un patient à une chimiothérapie pour un choriocarcinome. La nécessité d'obtenir un dosage extrêmement sensible et précis est par conséquent évidente. Malheureusement, chez un grand nombre de femmes, en particulier au cours de la ménopause, le taux de LH peut être tellement élevé qu'il donne des résultats faux positifs pour ce qui est de la teneur en hCG.

Exemple

On réduit un antisérum dirigé contre l'hCG (fabricant : Dakopatts) en fragments F(ab')₂ par une digestion à la pepsine et l'on lie ces fragments à du latex [latex F(ab')₂] suivant le procédé décrit dans le brevet des Etats-Unis d'Amérique US-A-4 397 960. Le latex sert donc de support aux fragments F(ab')₂ des anticorps, qui sont dirigés contre l'hCG. D'un autre côté, dans un dispositif d'analyse automatisé, on dilue 30 µl d'échantillon à analyser avec 300 µl de mélange de réaction à la pepsine (10 mg/ml de HCl 0,3 N) et on incube le mélange à la température ambiante pendant 5 minutes sous une agitation continue. On élève ensuite le pH du mélange à 7,5-8,0 par l'addition de 60 µl de Tris 1M. On ajoute encore à ce mélange 10 µl d'un antisérum de lapin anti-TSH (fraction d'IgG : 10 mg/ml) et une suspension à 0,05 % du latex recouvert de fragments F(ab')₂ préparé ci-dessus. Cet antisérum de lapin, source d'anticorps dirigés contre la chaîne alpha des hormones contenues dans l'échantillon, a été obtenu par injection intradermique deux fois par mois de 20 µg de TSH dans de l'adjuvant complet de Freund. On incube alors le mélange résultant à 37 °C pendant 35 minutes sous une agitation continue. On arrête la réaction par l'addition automatique de 1,0 ml de solution saline tamponnée par de la glycine contenant 0,1 % de Tween® et, après une nouvelle dilution de 20 fois, on compte les particules de latex non agglutinées. Le comptage des particules de latex non agglutinées donne en fait une mesure de la quantité de latex agglutiné qui, à son tour, permet de déterminer la quantité de hCG dans l'échantillon à analyser. On pourrait évidemment également mesurer directement la quantité de latex agglutinée. En dosant quatre concentrations différentes en hCG (5, 20, 50 et 200 mUI/ml), à raison de 10 fois chacune, on obtient la précision intrasérie suivante :

Précision intrasérie

| Conc. en hCG (mUI/ml) | D.S.[*] (mUI/ml) | C.V.[**], % |
|---|---|---|
| 6,53 | 0,46 | 7,0 |
| 19,45 | 0,49 | 2,5 |
| 41,20 | 1,13 | 2,7 |
| 149,20 | 8,32 | 5,6 |

En dosant également quatre concentrations différentes en hCG (10, 20, 50 et 200 mUI/ml), chaque jour pendant 15 jours, on obtient la précision intersérie suivante :

Précision intersérie

| Conc. en hCG (mUI/ml) | D.S.[*] (mUI/ml) | C.V.[**], % |
|---|---|---|
| 10,2 | 0,55 | 5,5 |
| 20,2 | 1,59 | 7,8 |
| 50,2 | 3,79 | 7,5 |
| 198,5 | 9,87 | 4,9 |

* D.S. = déviation standard ;
** C.V. = coefficient de variation.

Récupération

Si l'on ajoute à du sérum mâle, c'est-à-dire dépourvu d'hCG, 10, 50 et 200 mUI/ml d'hCG, les récupérations d'hCG sont respectivement de 87,9 %, 103,4 % et 103,4 %, résultats satisfaisants qui montrent que les autres hormones contenues dans le sérum n'ont pas interféré avec l'hCG.

La corrélation avec un radioimmunoessai effectué sur des échantillons cliniques a révélé un coefficient de corrélation de 0,96 avec une ligne de régression Y = — 11,2 + 1,06X pour 15 échantillons, dont la concentration variait entre 8 mUI/ml et 1 850 mUI/ml, ce qui est un résultat tout à fait satisfaisant.

Afin de tester l'efficacité du traitement avec l'antisérum anti-TSH, on a utilisé 10 sérums de femmes ménopausées qui, par radioimmunoessai (RIA), avaient montré des teneurs en LH très élevées, pour la mesure d'hCG.

| Teneur en LH(mUI/ml) telle que mesurée par RIA | Teneur en hCG sans anti-TSH(mUI/ml), telle que mesurée par PACIA | Teneur en hCG avec anti-TSH, telle que mesurée par PACIA |
|---|---|---|
| 117 | 22 | 9 |
| 74 | 12,5 | 3,5 |
| 232 | 25 | 10 |
| 77 | 15 | 6,5 |
| 62 | 11 | 3,5 |
| 74 | 7,2 | 1 |
| 82 | 9 | 6 |
| 77 | 21 | 7,4 |
| 91 | 24 | 9,2 |
| 135 | 13,2 | 1 |

Dans l'essai précité, on considère qu'une concentration en hCG de 25 mUI/ml est un résultat vrai positif, c'est-à-dire que, dans ce cas, la femme devrait être considérée comme étant enceinte. Comme on peut le voir d'après les résultats obtenus sans antisérum anti-TSH, au moins l'un des dix échantillons (le premier) aurait dû être considéré comme positif, tandis que deux autres de ces échantillons (22 et 24 mUI/ml) auraient probablement dû être rappelés et réanalysés, les résultats étant douteux. En fait, lorsque l'on dose la teneur en hCG des mêmes sérums avec incorporation d'anti-TSH suivant le procédé de l'invention, on peut voir qu'aucun échantillon n'est positif, toutes les valeurs d'hCG étant sensiblement inférieures à 25.

Il doit être entendu que la présente invention n'est en aucune façon limitée aux formes de réalisation ci-dessus et que bien des modifications peuvent y être apportées sans sortir du cadre du présent brevet.

## Revendications

1. Procédé de dosage immunologique d'une substance choisie dans un échantillon liquide contenant une ou plusieurs autres substances non choisies, susceptibles d'interférer dans le dosage de ladite substance, chacune de ces substances étant formée d'au moins deux chaînes ou fragments structuralement différents, l'une de ces chaînes ou l'un de ces fragments étant immunologiquement commun et l'autre immunologiquement distinct, caractérisé en ce qu'il comprend l'addition d'un agent dissociant choisi dans le groupe comprenant les agents protéolytiques, les agents rompant les ponts disulfure, les agents chaotropiques et les mélanges d'au moins deux de ces agents, audit échantillon liquide pour scinder la substance choisie à doser en au moins ses deux chaînes ou fragments, la ou les substances non choisies étant également scindées en au moins ses ou leurs deux chaînes ou fragments, le mélange du liquide ainsi obtenu, l'inactivation de l'agent dissociant, l'addition audit mélange d'un antisérum dirigé contre la chaîne ou le fragment immunologiquement commun de la substance choisie et de la ou des substances non choisies, l'addition à la solution résultante d'une suspension de particules recouvertes d'un antisérum dirigé contre la chaîne ou le fragment immunologiquement distinct de la substance choisie à doser et la détermination de la quantité de cette substance choisie dans l'échantillon liquide par une technique d'agglutination des particules précitées.

2. Procédé suivant la revendication 1, caractérisé en ce que la substance choisie et la ou les substances non choisies sont des hormones.

3. Procédé suivant la revendication 2, caractérisé en ce que lesdites substances sont des hormones choisies dans le groupe comprenant la thyréostimuline (TSH), l'hormone folliculostimulante (FSH), l'hormone lutéinisante (LH) et la gonadotropine chorionique humaine (hCG).

4. Procédé suivant la revendication 3, caractérisé en ce que la substance choisie à doser est la gonadotropine chorionique humaine.

5. Procédé suivant l'une quelconque des revendications 1 à 4, caractérisé en ce que les chaînes immunologiquement commune et distincte desdites hormones sont respectivement constituées par les chaînes alpha et bêta.

6. Procédé suivant l'une quelconque des revendications 1 à 5, caractérisé en ce que l'échantillon liquide est un liquide biologique.

7. Procédé suivant l'une quelconque des revendications 1 à 6, caractérisé en ce qu'après l'addition de l'agent dissociant, on mélange le liquide résultant pendant une durée de l'ordre de 5 à 15 minutes.

8. Procédé suivant l'une quelconque des revendications 1 à 7, caractérisé en ce que l'on inactive l'agent dissociant en élevant le pH du mélange à une valeur de l'ordre de 7 à 9.

9. Procédé suivant l'une quelconque des revendications 1 à 8, caractérisé en ce qu'après avoir ajouté les antisérums, on incube la solution ainsi obtenue sous agitation à une température d'environ 37 °C pendant une durée de l'ordre de 15 à 60 minutes.

10. Procédé suivant l'une quelconque des revendications 1 à 9, caractérisé en ce que l'agent dissociant est une enzyme protéolytique.

11. Procédé suivant la revendication 10, caractérisé en ce que l'enzyme protéolytique est choisie dans le groupe comprenant la pepsine, la papaïne et la trypsine.

12. Procédé suivant l'une quelconque des revendications 1 à 9, caractérisé en ce que l'agent dissociant est un agent rompant les ponts disulfure, tel que le mercaptoéthanol, le dithiotréitol.

13. Procédé suivant l'une quelconque des revendications 1 à 9, caractérisé en ce que l'agent dissociant est un agent chaotropique, tel que l'urée, le chlorhydrate de guanidine, le thiocyanate d'ammonium.

14. Procédé suivant l'une quelconque des revendications 1 à 13, caractérisé en ce que l'on détermine la quantité de substance choisie dans l'échantillon liquide par une technique d'agglutination de particules, en fonction du degré d'agglutination ou de non-agglutination de particules finement divisées.

15. Procédé suivant la revendication 14, caractérisé en ce que l'on utilise comme particules finement divisées des particules de latex ou des globules rouges.

16. Procédé suivant l'une ou l'autre des revendications 14 et 15, caractérisé en ce que la substance choisie est la gonadotropine chorionique humaine (hCG), l'antisérum dirigé contre la chaîne ou le fragment immunologiquement commun est un antisérum de lapin anti-TSH et l'antisérum dirigé contre la chaîne ou le fragment immunologiquement distinct est une suspension de latex recouvert de fragments F(ab')₂.

## Claims

1. A method of immunological assay of a chosen substance in a liquid sample containing one or more other non-chosen substances capable of interfering with the assay of the first-mentioned substance, each substance being made up of at least two structurally different chains or fragments, one chain or one fragment being immunologically common and the other immunologically distinct, the method being characterised in that it comprises adding a dissociating agent, chosen from the group comprising proteolytic agents, disulphide-bridge breaking agents, chaotropic agents and mixtures of at least two of these agents to the liquid sample in order to break the substance chosen for assay into at least its two chains or fragments, the non-chosen substance or substances being also broken into at least its or their two chains or fragments, and also comprises mixing the resulting liquid, inactivating the dissociating agent, adding to the mixture an antiserum directed against the chain or fragment immunologically common to the chosen substance and the non-chosen substance or substances, adding to the resulting solution a suspension of particles covered with an antiserum directed against the immunologically distinct chain or fragment in the substance chosen for assay, and determining the quantity of the chosen substance in the liquid sample by a technique of agglutinating the aforementioned particles.

2. A method according to claim 1, characterised in that the chosen substance and the non-chosen substance or substances are hormones.

3. A method according to claim 2, characterised in that the substances are hormones chosen from the group comprising thyreostimulin (TSH), the folliculostimulating hormone (FSH), the luteinising hormone (LH) and human chorionic gonadotropin (hCG).

4. A method according to claim 3, characterised in that the substance chosen for assay is human chorionic gonadotropin.

5. A method according to any of claims 1-4, characterised in that the immunologically common and the immunologically distinct chains in the aforementioned hormones are alpha and beta chains respectively.

6. A method according to any of claims 1 to 5, characterised in that the liquid sample is a biological liquid.

7. A method according to any of claims 1 to 6, characterised in that after addition of the dissociating agent, the resulting liquid is mixed for a time of about 5 to 15 minutes.

8. A method according to any of claims 1 to 7, characterised in that the dissociating agent is inactivated by raising the pH of the mixture to a value of about 7 to 9.

9. A method according to any of claims 1 to 8, characterised in that after adding the antisera, the resulting solution is incubated with agitation at a temperature of about 37 °C for a time of about 15 to 60 minutes.

10. A method according to any of claims 1 to 9, characterised in that the dissociating agent is a proteolytic enzyme.

11. A method according to claim 10, characterised in that the proteolytic enzyme is chosen from the group comprising pepsin, papain and trypsin.

12. A method according to any of claims 1 to 9, characterised in that the dissociating agent is a disulphide-bridge breaking agent such as mercaptoethanol or dithiothreitol.

13. A method according to any of claims 1 to 9, characterised in that the dissociating agent is a chaotropic agent such as urea or guanidine hydrochloride or ammonium thiocyanate.

14. A method according to any of claims 1 to 13, characterised in that the quantity of chosen substance in the liquid sample is determined by a particle agglutination technique, depending on the extent of agglutination or non-agglutination of finely divided particles.

15. A method according to claim 14, characterised in that the finely divided particles used are particles of latex or red corpuscles.

16. A method according to claim 14 or 15, characterised in that the chosen substance is human chorionic gonadotropin (hCG), the antiserum directed against the immunologically common chain or fragment is an anti-TSH rabbit serum and the antiserum directed against the immunologically distinct chain or fragment is a suspension of latex covered with F(ab')$_2$ fragments.


**Patentansprüche**

1. Immunologisches Bestimmungsverfahren einer ausgewählten Substanz in einer flüssigen Probe, die eine oder mehrere nichtausgewählte Substanzen enthält, welche mit der Bestimmung der genannten Substanz interferieren können, wobei jede der Substanzen aus wenigstens zwei struktural verschiedenen Ketten oder Fragmenten gebildet ist, wobei eine der Ketten oder eines der Fragmente immunologisch gleich und die andere bzw. das andere immunologisch verschieden ist, dadurch gekennzeichnet, daß es umfaßt die Zugabe eines Dissoziierungsmittels, ausgewählt aus einer Gruppe, umfassend proteolytische Mittel, disulfidbrückenspaltende Mittel, chaotropische Mittel und eine Mischung aus wenigstens zwei dieser Mittel, die genannte flüssige Probe zum Spalten der ausgewählten, zu bestimmenden Substanz in wenigstens seine zwei Ketten oder Fragmente, wobei die nichtausgewählte Substanz oder die nichtausgewählten Substanzen ebenfalls in wenigstens seine zwei oder ihre zwei Ketten oder Fragmente gespalten werden, das Mischen der so erhaltenen Flüssigkeit, die Inaktivierung des Dissoziierungsmittels, die Zugabe eines Antiserums zur Mischung, das gegen die Kette oder das Fragment gerichtet ist, die immunologisch gleich zur ausgewählten Substanz und zu der oder zu den nichtausgewählten Substanzen sind, die Zugabe einer Suspension von Partikeln, die von einem Antiserum bedeckt sind, das gerichtet ist gegen die Kette oder das Fragment, die immunologisch unterschiedlich sind zur ausgewählten, zu bestimmenden Substanz und die Bestimmung der Menge der ausgewählten Substanz in der flüssigen Probe mittels Agglutination der vorgenannten Partikel.

2. Verfahren nach Anspruch 1, dadurch gekennzeichnet, daß die ausgewählte Substanz und die nichtausgewählte Substanz oder die nichtausgewählten Substanzen Hormone sind.

3. Verfahren nach Anspruch 2, dadurch gekennzeichnet, daß die genannten Substanzen Hormone sind, ausgewählt aus einer Gruppe, umfassend Thyreotropes Hormon (TSH), Follikelstimulierendes Hormon (FSH), Luteinisierungshormon (LH) und menschliches Choriongonadotropin (hCG).

4. Verfahren nach Anspruch 3, dadurch gekennzeichnet, daß die ausgewählte, zu bestimmende Substanz menschliches Choriongonadotropin ist.

5. Verfahren nach einem der Ansprüche 1-4, dadurch gekennzeichnet, daß die Ketten, die immunologisch gleich oder unterschiedlich zu den genannten Hormonen sind, jeweils durch die Alphaketten und Betaketten konstituiert sind.

6. Verfahren nach einem der Ansprüche 1-5, dadurch gekennzeichnet, daß die flüssige Probe eine biologische Flüssigkeit ist.

7. Verfahren nach einem der Ansprüche 1-6, dadurch gekennzeichnet, daß man nach Zugabe des Dissoziierungsmittels die resultierende Flüssigkeit während einer Dauer der Größenordnung von 5 bis 15 Minuten mischt.

8. Verfahren nach einem der Ansprüche 1-7, dadurch gekennzeichnet, daß man das Dissoziierungsmittel durch Erhöhen des pH der Mischung auf einen Wert in der Größenordnung von 7 bis 9 inaktiviert.

9. Verfahren nach einem der Ansprüche 1-8, dadurch gekennzeichnet, daß man nach Zugabe der Antiseren die so erhaltene Lösung unter Rühren bei einer Umgebungstemperatur von 37 °C während einer Dauer in der Größenordnung von 15 bis 60 Minuten inkubiert.

10. Verfahren nach einem der Ansprüche 1-9, dadurch gekennzeichnet, daß das Dissoziierungsmittel ein proteolytisches Enzym ist.

11. Verfahren nach Anspruch 10, dadurch gekennzeichnet, daß das proteolytische Enzym ausgewählt ist aus einer Gruppe umfassend Pepsin, Papain und Trypsin.

12. Verfahren nach einem der Ansprüche 1-9, dadurch gekennzeichnet, daß das Dissoziierungsmittel ein disulfidbrückenspaltendes Mittel, wie Mercaptoethanol, Dithiotreitol ist.

13. Verfahren nach einem der Ansprüche 1-9, dadurch gekennzeichnet, daß das Dissoziierungsmittel ein chaotropisches Mittel, wie Harnstoff, Guanidinchlorhydrat, Ammoniumthiocyanat ist.

14. Verfahren nach einem der Ansprüche 1-13, dadurch gekennzeichnet, daß man die Menge der ausgewählten Substanz in der flüssigen Probe bestimmt durch eine Partikelbindetechnik, in Abhängigkeit vom Grad der Agglutination oder der Nicht-Agglutination der feinverteilten Partikel.

15. Verfahren nach Anspruch 14, dadurch gekennzeichnet, daß man als feinverteilte Partikel Latex oder rote Kügelchen verwendet.

16. Verfahren nach Anspruch 14 oder 15, dadurch gekennzeichnet, daß die ausgewählte Substanz das Choriongonadotropin, das Antiserum, gerichtet gegen die Kette oder das immunologisch gleiche Fragment ein Antiserum vom Kaninchenanti-TSH ist und das Antiserum, gerichtet gegen die immunologisch unterschiedliche Kette oder das Fragment eine Latex-Suspension, bedeckt mit Fragmenten des F(ab')$_2$ ist.